Europäisches Patentamt

(19) European Patent Office   (11) Numéro de publication: **0 097 664**
**B1**
Office européen des brevets

(12) **FASCICULE DE BREVET EUROPEEN**

(45) Date de publication du fascicule du brevet:
12.03.86

(51) Int. Cl.⁴: **C 12 Q 1/68**

(21) Numéro de dépôt: **83900047.8**

(22) Date de dépôt: **23.12.82**

(86) Numéro de dépôt international:
**PCT/FR 82/00220**

(87) Numéro de publication internationale:
**WO 83/02286 (07.07.83 Gazette 83/16)**

(54) **PROCEDE FAISANT INTERVENIR UNE SONDE CONTENANT UN ACIDE NUCLEIQUE MODIFIE ET RECONNAISSABLE PAR DES ANTICORPS SPECIFIQUES ET PERMETTANT DE DETECTER ET DE CARACTERISER UNE SEQUENCE D'ADN HOMOLOGUE.**

(30) Priorité: **23.12.81 FR 8124131**

(43) Date de publication de la demande:
**11.01.84 Bulletin 84/2**

⑤ Mention de la délivranc du brevet:
**12.03.86 Bulletin 86/11**

(84) Etats contractants désignés:
**BE CH DE GB LI**

(73) Titulaire: **INSTITUT PASTEUR, 25-28, rue du Docteur Roux, F-75724 Paris Cedex 15 (FR)**

(72) Inventeur: **TCHEN, Paul, 18, rue du Télégraphe, F-92000 Nanterre (FR)**
Inventeur: **CAMI, Anne Brigitte, 48, rue Paul Barruel, F-75015 Paris (FR)**
Inventeur: **LENG, Marc, 50, rue de la Racinerie, F-45590 St Cyr en Val (FR)**
Inventeur: **KOURILSKY, Philippe, 207, rue de Vaugirard, F-75015 Paris (FR)**

(74) Mandataire: **Gutmann, Ernest et al, S.C. Ernest Gutmann - Yves Plasseraud 67, boulevard Haussmann, F-75008 Paris (FR)**

(56) Documents cités:
**GB - A - 2 019 408**
**US - A - 4 358 535**

Biochemistry, vol. 18, no. 7, publié en 1979, Am. Chem. Soc. (Easton, Pa., US) E. Sage et al.: "Reactivity of the antibodies to DNA modified by the carcinogen N-acetoxy-N-acetyl-2-aminofluorene", voir pages 1328-1332
Chemical Abstracts, vol. 94, no. 13, publié le 30 mars 1981 (Columbus, Ohio, US) M. Spodheim-Maurizot et al. "Antibodies to N-hydroxy-2-aminofluorene modified DNA as probes in the study of DNA reacted with derivatives of 2-acetylamino-fluorene", voir page 232, colonne 2, l'abrégé no. 97632j, Carcinogenesis, 1980, 807-12 (Eng.)
Chemical Abstracts, vol. 89, no. 21, publié le 20 novembre 1978 (Columbus, Ohio, US) M. Leng et al.: "Antibodies to DNA modified by the carcinogen

(56) Documents cités: (suite)
N-acetoxy-N-2-acetyl-aminofluorene", voir page 166, colonne 1, l'abrégé no. 174795r, FEBS Lett., 1978, 207-210 (Eng.)
Chemical Abstracts, vol. 91, no. 5, publié le 30 juillet 1979 (Columbus, Ohio, US) M. Guignes et al.: "Reactivity of antibodies to guanosine modified by the carcinogen N-acetoxy-N-2-acetylaminofluorene", voir page 150, colonne 1, l'abrégé no. 33858t, Nucleic Acids Res., 1979, 733-744 (Eng.)
Chemical Abstracts, vol. 92, no. 17, publié le 28 avril 1980 (Columbus, Ohio, US) G. de Murcia et al.: "Electron microscopic visualization of N-acetoxy-N-2-acetylaminofluorene binding sites in C01E1 DNA by means of specific antibodies", voir page 129, colonne 2, abrégé no. 141501a, Proc. Natl. Acad. Sci. USA, 1979, 6076-80 (Eng.)

ACTORUM AG

## Description

L'invention est relative à l'utilisation d'une sonde pour détecter et caractériser une séquence d'ADN déterminée dans un échantillon susceptible de le contenir, ladite sonde contenant un acide nucléique modifié homologue de la susdite séquence d'ADN déterminée et reconnaissable par des anticorps spécifiques. Plus particulièrement, l'invention concerne une sonde modifiée chimiquement de telle sorte qu'elle puisse, après hybridation avec la séquence d'ADN homologue recherchée, être détectée par des anticorps spécifiques à l'égard de la sonde elle-même.

Une sonde et un procédé mettant en œuvre un procédé de ce type ont déjà été décrits dans le brevet anglais N° 2019408. Conformément à l'un des modes de réalisations de la technique décrite dans ce brevet, la sonde est constituée par un acide nucléique contenant une séquence homologue de la séquence recherchée au sein de l'échantillon étudié, cette sonde étant en outre modifiée par au moins un groupe chimique permettant ensuite le couplage de la sonde avec une enzyme. Le procédé décrit comprend alors, dans l'une de ses formes préférées d'exécution, la mise en contact de cette sonde avec une composition d'acides nucléiques présumée contenir la séquence recherchée, dans des conditions autorisant, après dénaturation, l'hybridation éventuelle entre la sonde et la séquence recherchée, l'élimination de l'excès de sonde et le couplage du produit d'hybridation entre la sonde et la séquence recherchée avec l'enzyme, la présence de la séquence recherchée dans la composition initiale étant révélée par l'action du produit résultant du susdit couplage sur un substrat spécifique de l'enzyme. Selon le brevet, il va donc de soi que le groupe chimique utilisé pour la modification de la sonde d'acide nucléique doit être tel qu'il n'interfère pas avec les capacités d'hybridation entre la séquence recherchée éventuellement contenue dans la composition étudiée et la séquence homologue de la sonde ainsi modifiée.

Par ailleurs, il est connu que des ADN sont susceptibles de réagir dans des conditions appropriées avec des substances carcinogènes, telles que le N-acétoxy-N-2-acétylaminofluorène, pour former un produit susceptible d'être reconnu par des anticorps formés, d'une part, contre le N-2-(guanosine-8-yl)-acétaminofluorène et, d'autre part, contre les mêmes ADN modifiés par le N-acétoxy-N-acétylaminofluorène. Ces techniques ont notamment été décrites dans un article de Gilbert de Murcia et coll., intitulé «Visualisation par microscopie électronique des sites de fixation du N-acétoxy-N-2-acétylaminofluorène sur un ADN de ColE 1 au moyen d'anticorps spécifiques» («Proc. Natl. Acad. Sci. USA»), tome 76, N° 12, 6076-6080, déc. 1979).

Dans les conditions décrites par ces auteurs, il est possible de modifier de 0,07 à 0,15% des bases de l'ADN traité par le N-acétoxy-N-2-acétylaminofluorène, les points de fixation de cette dernière substance chimique sur l'ADN pouvant ensuite être repérés par microscopie électronique, après réaction préalable de l'ADN ainsi modifié avec des anticorps du genre sus-indiqué préalablement formés chez le lapin, puis avec des anti-immunoglobulines de lapins marqués à la ferritine. La technique décrite permet par conséquent de distinguer des ADN natifs sains et des ADN ayant été soumis à l'action de substances carcinogènes.

Les conditions dans lesquelles les susdits anticorps reconnaissent des ADN modifiés par le N-acétoxy-N-2-acétylaminofluorène, et l'identification des groupes immunodéterminants mis en jeu dans cette reconnaissance ont fait l'objet de l'article intitulé «Réactivité des anticorps à l'égard d'ADN modifié par le carcinogène N-acétoxy-N-acétyl-2-aminofluorène» de E. Sage et coll., «Biochemistry» (1979), vol. 18, N° 7, 1328-1332. L'étude a pris son essor à partir de la constatation faite antérieurement par d'autres auteurs que la géométrie de la double hélice était modifiée comme suite à la réaction du carcinogène sur le C(8) des résidus guanine modifiés et qu'il en résultait une distorsion de conformation de l'acide nucléique. Sage et coll. ont eux-mêmes exprimé l'hypothèse que les noyaux fluorène sont empilés à proximité des bases voisines, que les résidus guanine concernés étaient extérieurs à la double hélice et qu'un certain nombre de paires de bases étaient rompues.

L'invention repose sur la découverte que la modification d'une séquence d'ADN par le N-acétoxy-N-2-acétylaminofluorène n'altérait pas, après dénaturation préalable de cet ADN modifié, sa capacité de s'hybrider avec une séquence complémentaire d'ADN ne portant pas de tels groupes de modification, lorsque ces séquences sont placées dans des conditions permettant une telle hybridation. L'invention tire profit de cette découverte pour proposer un procédé perfectionné de détection de la présence éventuelle et de la caractérisation d'une séquence ou d'un fragment déterminé d'acide nucléique, notamment d'un gène au sein d'une composition susceptible de le contenir.

Le procédé selon l'invention est caractérisé en ce que l'on met en contact avec la composition présumée contenir une séquence ou un fragment déterminé d'acide nucléique, une sonde contenant un acide nucléique complémentaire susceptible de s'hybrider avec la séquence d'acide nucléique ou le gène recherché, la sonde étant plus particulièrement caractérisée en ce qu'elle porte au moins un groupe N-2-acétylaminofluorène fixé de façon covalente à au moins l'une des bases de cette sonde, l'éventuelle présence de la séquence d'acide nucléique ou de gène recherché étant ensuite révélable par action d'anticorps efficaces vis-à-vis de la N-2-(guanosine-8-yl)-acétylaminofluorène ou préalablement préparés vis-à-vis de la sonde portant des résidus d'acétylaminofluorène (ci-après dénommés DNA-AAF).

Naturellement, il va de soi que le DNA-AAF utilisé comme sonde est mis en présence de l'ADN à étudier dans des conditions permettant le réappariement de séquences complémentaires, ce qui implique naturellement une dénaturation préalable dans des conditions bien connues des ADN susceptibles de s'hybrider mutuellement.

Après hybridation, l'ADN-AAF non hybridé de façon spécifique est de préférence éliminé par rinçage avant que l'on ne procède à la détection des hybrides formés, notamment par leur mise en présence avec des anticorps anti-DNA-AAF, qui peuvent alors se fixer sur la sonde à la fois modifiée et hybridée avec la séquence d'ADN recherchée, lorsque celle-ci était présente dans la composition utilisée.

Après rinçage des anticorps excédentaires encore présents, les anticorps fixés peuvent être, soit précipités, soit révélés.

De préférence, la révélation est faite au moyen d'un anticorps anti-DNA-AAF, avantageusement marqué par une enzyme dont on peut ensuite détecter ou doser l'activité vis-à-vis d'un substrat spécifique. Avantageusement, on utilisera celles des enzymes qui sont susceptibles d'induire une réaction colorée au niveau des substrats correspondants.

La révélation à l'aide d'enzymes donnant des réactions colorées est très rapide.

La méthode est très sensible, surtout si on utilise des systèmes amplificateurs (chapelets, arbres ou boules d'anticorps associés à des enzymes), de sorte qu'elle permet de localiser des gènes après hybridation *in situ* sur des chromosomes, par exemple dans le cas de diagnostics prénatals.

La méthode peut être quantitative, par mesure de l'intensité de la coloration.

Des caractéristiques supplémentaires de l'invention apparaîtront encore au cours de la description qui suit d'un exemple type de mise en œuvre du procédé selon l'invention.

On a fait usage des matières et méthodes suivantes:

### Les ADN:

— ADN de phage pBR 322 portant une séquence de gène de ribosome de hamster de 6,6 kbar insérée au sit EcoRI (clone PWE 6);

— ADN distinct de phage λ 57 comme témoin négatif.

### L'ADN traité à l'AAF (DNA-AAF)

De l'ADN du clone PWE 6 a été linéarisé (par l'enzyme de restriction Sal I) et traité à l'AAF selon la technique décrite par G. de Murcia *et al.* (PNAS vol. 76, N° 12, p. 6076-6080, 1979). Le nombre des guanines modifiées a été estimé à 2% du nombre de paires de bases par mesure de la densité optique à 305 nm et 260 nm.

### Les anticorps:

— sérum DNA-AAF obtenu par immunisation d'un lapin;

— anticorps anti-Guo-AAF de lapin purifié sur colonne d'affinité;

— anticorps de chèvre anti-IgG de lapin liés à de la peroxydase.

Les anticorps ont été obtenus dans les conditions décrites dans l'article susdit.

### Essai de détection du DNA-AAF

Des quantités variables de DNA-AAF ont été déposées sur des filtres de nitrocellulose (Schleicher et Schüll, type BA 85) de 5 mm de diamètre.

L'ADN a préalablement été dilué dans une solution 2 × SSC et dénaturé à 100° C, pendant 5 min.

Après dépôt, les membranes ont été mises au four à 80° C pendant 2 h.

Les membranes ont ensuite été traitées avec une solution 3% albumine bovine (SIGMA réf. 7888), 1 SSC, à 40° C pendant 1 h, puis incubées 30 min à température ambiante dans la même solution en présence d'anticorps anti-DNA-AAF ou anti-Guo-AAF de lapin à 2 μg/ml final.

Après incubation, les membranes ont été lavées 7 fois avec du PBS, à température ambiante, puis mises à incuber 30 min dans une solution 3% albumine bovine, 1 SSC contenant des anticorps de chèvre anti-IgG de lapin liés à de la peroxydase à 2 μg/ml final.

Après lavage 7 fois avec du PBS, la réaction colorée a été faite par addition de la solution suivante, préparée extemporanément:

— 2 mg de 3-amino-9-éthylcarbazol (SIGMA réf. A 5754) dissous dans 0,5 ml de N-N'-diméthylformamide;

— 9,5 ml de tampon acétate acétique 0,05M, pH 5,1;

— 10 μl d'$H_2O_2$ (Merk réf. 7209).

### Test d'hybridation avec du DNA-AAF utilisé comme sonde

Dépôt de quantités variables de DNA-PWE 6:
1. 100 ng
2. 10 ng
3. 1 ng
4. 100 pg

Après dépôt, les filtres sont mis à 80° C pendant 2 h, puis préhybridés 4 h à 68° C dans une solution 6 × SSC et 10 × Denhardt.
(1 × Denhardt contenant:

0,02% de polyvinyl pyrrolidone,

0,02% du réactif commercialisé sous la désignation Ficolle 400 par la société «Pharmacia fine Chemicals»,

0,02% d'albumine bovine.)

Ils sont ensuite hybridés dans une solution 2 × SSC 1 × Denhardt en présence de 200 μl par membrane de solution de DNA-AAF préalablement dénaturé contenant respectivement:

10 ng/ml final
1 ng/ml final et
100 pg/ml final

Après hybridation, les filtres ont été lavés
30 min dans 2 × SSC 1 × Denhardt
30 min dans 1 × SSC 1 × Denhardt
30 min dans 0,5 × SSC 1 × Denhardt
30 min dans 0,2 × SSC 1 × Denhardt
1 h dans 0,1 × SSC 1 × Denhardt
puis incubés pendant 1 h à 40° C dans une solution contenant 3% d'albumine à 1 × SSC. La suite des opérations a été faite comme précédemment (mise en présence avec des anticorps anti-DNA-AAF ou anti-Guo-AAF, lavage PBS, anticorps + peroxydase, lavage PBS et révélation).

Après révélation on observe des taches colorées dont l'intensité (plus forte pour les concentrations élevées, plus faible pour des concentrations basses d'ADN) dépend de la quantité d'ADN hybridé.

La méthode de détection sus-indiquée a conduit à des résultats entièrement négatifs au terme d'essais d'hybridation réalisés entre le témoin négatif (utilisé en des quantités atteignant jusqu'à 90 ng) et le DNA-AAF.

Au titre des variantes utilisables au niveau de la détection des hybrides formés avec la sonde selon l'invention, on citera:

— la révélation des hybrides formés par la radioactivité, par exemple grâce à l'utilisation d'anticorps anti-DNA-AAF rendus radioactifs par de l'iode 125 ou 131 ou de protéine A radioactive, qui va se fixer sur les anticorps.

Enfin, on citera l'application de la sonde selon l'invention à la purification d'un ADN complémentaire contenu dans une composition initiale, notamment au moyen

— de protéine A associée à un support solide (par exemple constitué de billes d'agarose),

— d'anticorps précipitants associés ou non à un support solide (billes d'agarose, de latex, etc.), pour assurer la précipitation sélective de l'hybride formé.

## Revendications

1. Procédé de détection de la présence d'une séquence d'acide nucléique déterminée, d'un gène ou d'un fragment de gène recherché dans une composition ou échantillon présumé la contenir, comprenant la mise en contact de cette composition avec une sonde d'acide nucléique contenant une séquence complémentaire ou homologue de la séquence d'acide nucléique déterminée, du gène ou fragment de gène recherché, dans des conditions permettant, après dénaturation, l'hybridation, entre la séquence déterminée, le gène ou fragment de gène recherché, d'une part, et la séquence complémentaire de ladite sonde, d'autre part, et l'élimination de la sonde non hybridée, caractérisé en ce que ladite sonde porte au moins un groupe N-2-acétylaminofluorène fixé de façon covalente à au moins l'une des bases de cette sonde, l'éventuelle présence de la séquence d'acide nucléique, du gène ou fragment de gène déterminé étant ensuite révélable par action d'anticorps efficaces contre la sonde portant les résidus d'acétylaminofluorène.

2. Procédé selon la revendication 1, caractérisé en ce que la sonde comporte des guanines modifiées par le N-acétoxy-N-2-acétylaminofluorène et que la révélation de la séquence d'acide nucléique, du gène ou fragment de gène recherché est faite par l'intermédiaire d'anticorps efficaces contre le N-2-(guanosine-8-yl)-acétylaminofluorène ou contre la sonde portant les résidus d'acétylaminofluorène.

3. Procédé pour la purification d'une séquence d'acide nucléique déterminée, d'un gène ou d'un fragment de gène recherché dans une composition ou échantillon présumé la contenir, comprenant la mise en contat de cette composition avec une sonde d'acide nucléique contenant une séquence complémentaire ou homologue de la séquence d'acide nucléique déterminée, du gène ou fragment de gène recherché, dans des conditions permettant, après dénaturation, l'hybridation, entre la séquence déterminée, le gène ou fragment de gène recherché, d'une part, et la séquence complémentaire de ladite sonde, d'autre part, et l'élimination de la sonde non hybridée, caractérisé en ce que ladite sonde porte au moins un groupe N-2-acétylaminofluorène fixé de façon covalente à au moins l'une des bases de cette sonde, et en ce que l'on procède à la séparation de l'hybride formé par réaction avec une protéine A fixée à un support solide ou par précipitation sélective de l'hybride avec des anticorps précipitants associés ou non à un support solide.

4. Procédé selon la revendication 3, caractérisé en ce que la sonde comporte des guanines modifiées par le N-2-(guanosine-8-yl)-acétylaminofluorène.

## Patentansprüche

1. Verfahren zum Nachweis einer vorhandenen bestimmten Nukleinsäuresequenz, eines in einer Zusammensetzung bzw. Probe zu ermittelnden Gens oder Genfragments, in welcher es mutmasslich vorhanden ist; das Verfahren schliesst das Zusammenkommen der Zusammensetzung mit einer Nukleinsäuresonde ein, die eine komplementäre oder homologe Sequenz der bestimmten Nukleinsäuresequenz des zu ermittelnden Gens bzw. Genfragments enthält, und dies unter Bedingungen, die nach Denaturation die Hybridisierung zwischen der bestimmten Sequenz und dem zu ermittelnden Gen bzw. Genfragment einerseits, und der komplementären Sequenz der besagten Sonde anderseits sowie den Ausschluss der nicht hybridisierten Sonde ermöglichen; das Verfahren ist dadurch gekennzeichnet, dass die besagte Sonde wenigstens eine Gruppe N-2-Azetylaminofluoren trägt, die auf konvalenter Art wenigstens an einer der Sondenbasis gebunden ist, wobei das eventuelle Vorhandensein der Nukleinsäuresequenz des zu ermittelnden Gens bzw. Genfragments, anschliessend durch Einwirkung der Antikörper nachweisbar ist, die wirksam gegen die Sonde sind, die Azetylaminofluorenrückstände trägt.

2. Das Verfahren gemäss Anspruch 1 ist dadurch gekennzeichnet, dass die Sonde Guanine trägt, die vom N-Azetoxi-N-2-Azetylaminofluoren abgewandelt sind, und dadurch, dass der Nachweis der Nukleinsäuresequenz des zu ermittelnden Gens bzw. Genfragments mittels Antikörper erfolgt, die wirksam gegen das N-2-(Guanosin-8-yl)aceztylaminofluoren oder gegen die Sonde sind, welche Azetylaminofluorenrückstände trägt.

3. Das Verfahren zur Reinigung einer bestimmten Nukleinsäuresequenz, eines in einer Zusammensetzung oder Probe vermutlich enthaltenen und zu ermittelnden Gens oder Genfragments, schliesst das Inkontaktkommen der Zusammensetzung mit einer Nukleinsäuresonde ein, die eine komplementäre oder homologe Sequenz der bestimmten Nukleinsäuresequenz des zu ermittelnden Gens oder Genfragments enthält, und wird unter Bedingungen ausgeführt, die nach Denaturation die Hybridisierung zwischen der bestimmten Sequenz, dem zu ermittelnden Gen oder Genfragment einerseits, und der komplementären Sequenz der besagten Sonde anderseits, sowie den Ausschluss der nicht hybridisierten Sonde erlauben; das Verfahren ist dadurch gekennzeichnet, dass die besagte Sonde wenigstens eine Gruppe N-2-Azetylaminofluoren trägt, die auf konvalenter Art an wenigstens eine der Basis dieser Sonde gebunden ist, und dadurch, dass die Absonderung der gebildeten Hybride durch Reaktion mit einem an einem festen Träger gebundenen A-Protein oder durch selektive Fällung der Hybride mittels fällenden Antikörper – die an einem festen Träger gebunden oder nicht gebunden sind – vorgenommen wird.

4. Das Verfahren gemäss Anspruch 3 wird dadurch gekennzeichnet, dass die Sonde Guanine trägt, die von dem N-2-(Guanosine-8-yl)azetylaminofluoren abgewandelt sind.

## Claims

1. A method for detecting the presence of a given nucleic acid sequence, of a gene or a gene fragment sought in a composition or sample which is supposed to contain it, comprising contacting said composition with a probe of nucleic acid containing a sequence complementary, or homologous, to the given nucleic acid sequence of the gene or gene fragment sought under conditions enabling, after denaturation, hybridization between the given sequence, the gene or gene fragment sought, on the one hand, and the sequence complementary to said probe on the other hand, and eliminating the non hybridized probe, wherein said probe bears at least one N-2-actylaminofluorene group fixed covalently to at least one of the bases of the said probe, the possible presence of the nucleic acid sequence, of the gene or gene fragment sought being then revealable by the action of antibodies effective with respect to the probe bearing the acetylaminofluorene residues.

2. A method according to Claim 1, wherein said probe comprises guanines modified by N-acetoxy-N-2-acetylaminofluorene and that the nucleic acid sequence of the gene or gene fragment sought is shown by using effective antibodies with respect to N-2-(guanine-8-yl)-acetylaminofluorene or with respect to the probe bearing acetylaminofluorene residues.

3. A process for purifying a given nucleic acid sequence, of a gene or a gene fragment sought in a composition or sample which supposedly contains the same, comprising contacting said composition with a nucleic acid probe containing a sequence complementary, or homologous, to the given nucleic acid sequence of the gene or the gene fragment sought under conditions enabling hybridization, after denaturation, between the given sequence and the gene or gene fragment sought, on the one hand, and the sequence complementary to said probe on the other hand, and eliminating the non hybridized probe, wherein said probe bears at least one N-2-acetylaminofluorene group covalently bound to at least one of the bases of said probe and that the separation of the hybrid formed is brought about by reaction with a protein A fixed to a solid support or by selective precipitation of the hybrid with precipitating antibodies associated or not to a solid support.

4. A method according to Claim 3, wherein the probe comprises guanines modified by N-2-(guanosine-8-yl)-acetylaminofluorene.